# EUROPEAN PATENT SPECIFICATION

(11) **EP 4 008 285 B1**
(45) Date of publication and mention of the grant of the patent: **14.05.2025**
(21) Application number: 20853556.7
(22) Date of filing: 29.10.2020
(51) Int. Cl.: A61B 18/18, A45D 26/00, A61N 5/06, A61B 18/20

(54) **HAIR REMOVAL DEVICE**
DEPILATOR
DISPOSITIF D'ÉPILATION

(30) Priority: 21.10.2020 CN 202011134930; 21.10.2020 CN 202022365212 U
(43) Date of publication of application: 08.06.2022
(73) Proprietor: Shenzhen Ulike Smart Electronics Co., Ltd., Shenzhen, Guangdong 518000 (CN)
(72) Inventor: PAN, Yuping, Shenzhen Guangdong 518000 (CN); LI, Xiang, Shenzhen Guangdong 518000 (CN)
(74) Representative: Zaboliene, Reda
(86) International application number: PCT/CN2020/124733
(87) International publication number: WO 2022/082837

(56) References cited:
- EP-B1- 3 263 177
- WO-A1-2015/098427
- CN-A- 111 700 725
- CN-A- 111 700 725
- CN-A- 111 743 622
- CN-A- 111 743 622
- CN-U- 209 464 502
- CN-U- 211 534 779
- CN-U- 211 674 531
- CN-U- 211 674 531
- US-A1- 2012 165 682

## Description

### TECHNICAL FIELD

The present disclosure relates to a field of electronic devices, and in particular to a depilator.

### BACKGROUND

At present, the depilator uses laser to irradiate hair follicles of a skin of a user to achieve hair removal. However, the depilator can only achieve a hair removal function, and cannot care the skin of the user, resulting in a single function of the current depilator.

### SUMMARY OF THE DISCLOSURE

The present disclosure provides a multifunctional and highly experienced depilator.

The present disclosure provides a depilator. The depilator includes a head portion and a hand-held portion coupled to the head portion. The head portion includes a cold compressing portion and an illuminating portion arranged around the cold compressing portion. The illuminating portion is configured to emit light for caring a skin of a user. The hand-held portion includes a hair removal device, a heat conducting portion, and a cool driving portion. The hair removal device is directly opposite to the cold compressing portion, to emit light to the cold compressing portion. The heat conducting portion is in contact with the cold compressing portion, to absorb heat of the cold compressing portion. The cool driving portion is fixed on one side of the hair removal device away from the cold compressing portion, and is configured to drive external cooling medium to flow through a part of the heat conducting portion away from the cold compressing portion, so that the heat of the heat conducting portion is taken away by the external cooling medium.

In the depilator provided by the present disclosure, the head portion includes the cold compressing portion and the illuminating portion arranged around the cold compressing portion. The illuminating portion is configured to emit light for caring the skin of the user. The cold compressing portion is directly opposite to the cold compressing portion, and is configured to emit light to the cold compressing portion, so that the cold compressing portion can emit light to irradiate hair follicles of the skin of the user, and use the light to penetrate the skin to irradiate the hair follicles for hair removal, so that the depilator has functions of hair removal and skin care.

The Chinese patent application No. CN 211674531U discloses a depilator, in particular to a portable depilator. The portable hair removal instrument comprises a head part and a shell connected with one end of the head part, wherein an emitter capable of emitting light rays is arranged in the shell, the light rays emitted by the emitter act on the skin for hair removal after passing through the head, the head is at least provided with a phototherapy lamp, and the light rays emitted by the phototherapy lamp act on the skin through the head and nurse the skin. Theportable unhairing instrument has the advantages of being rich in function, good in skin care effect and the like.

The Chinese patent application No. CN 111743622A discloses a portable unhairing instrument that includes a shell, a cold compress part, a heat conduction plate, an unhairing device and a control device, wherein the shell is provided with a head part, a tail part opposite to the head part and a peripheral side part connected between the head part and the tail part; the shell is provided with a cooling inlet, a first cooling outlet, a second cooling outlet and a third cooling outlet; the cooling inlet, the first cooling outlet and the third cooling outlet are formed in the peripheral side part; the cooling inlet and the first cooling outlet are respectively positioned on two adjacent side surfaces; the cooling inlet and the third cooling outlet are located in the same side face, the second cooling outlet is formed in the tail, the heat conduction plate is close to the cooling inlet and the third cooling outlet, the unhairing device right faces the cold compress part and is close to the first cooling outlet, and the control device is located on the side, away from the cold compress part, of the unhairing device and is close to the second cooling outlet. The internal temperature of the body of the portable unhairing instrument is integrally and quickly reduced, and the safety of the portable unhairing instrument is ensured.

### Summary of the invention

The invention is defined in appended independent claim 1, further embodiments are described in the dependent claims.

### BRIEF DESCRIPTION OF THE DRAWINGS

In order to explain technical solutions of the embodiments of the present disclosure more clearly, the following will briefly introduce the drawings that need to be used in the embodiments. Obviously, the drawings in the following description are some embodiments of the present disclosure. For those of ordinary skill in the art, other drawings can be obtained based on these drawings without creative work.
FIG. 1 is an exploded schematic diagram of a depilator provided by one embodiment of the present disclosure;
FIG. 2 is an assembly schematic diagram of the depilator provided by one embodiment of the present disclosure;
FIG. 3 is another exploded schematic diagram of the depilator provided by one embodiment of the present disclosure;
FIG. 4 is a partial schematic diagram of the depilator provided by one embodiment of the present disclosure;
FIG. 5 is a partial exploded schematic diagram of the depilator provided by one embodiment of the present disclosure;
FIG. 6 is another exploded schematic diagram of the depilator provided by one embodiment of the present disclosure.

### DETAILED DESCRIPTION

The technical solutions in the embodiments of the present disclosure will be described clearly and completely with reference to the accompanying drawings in the embodiments of the present disclosure. Obviously, the described embodiments are only a part of the embodiments of the present disclosure, but not all the embodiments. All other embodiments obtained by a person of ordinary skill in the art based on the embodiments in the present disclosure without creative work shall fall within a protection scope of the present disclosure.

In the description of the embodiments of the present disclosure, it should be understood that an orientation or positional relationship indicated by a term "thickness" is based on an orientation or positional relationship shown in the drawings, which is only for convenience of describing the present disclosure and simplifying the description, and does not imply or point that the device or element must have a specific orientation, be constructed and operated in a specific orientation, and therefore cannot be understood as a limitation of the present disclosure.

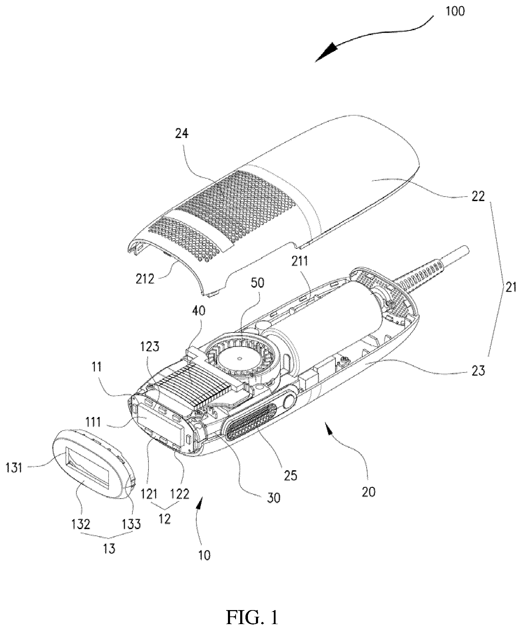

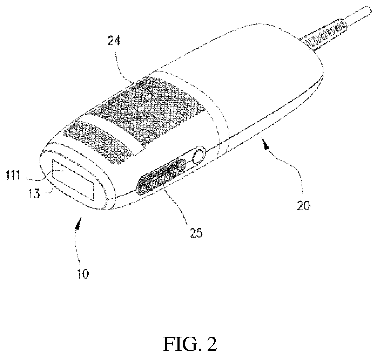

Please referring to FIGS. 1 and 2 , the present disclosure provides a depilator 100. The depilator 100 includes a head portion 10 and a hand-held portion 20 coupled to the head portion 10. The head portion 10 includes a cold compressing portion 11 and an illuminating portion 12 arranged around the cold compressing portion 11. The illuminating portion 12 is configured to emit light for caring a skin of a user. The hand-held portion 20 includes a hair removal device 30, a heat conducting portion 40 and a cool driving portion 50. The hair removal device 30 is directly opposite to the cold compressing portion 11, and is configured for emitting light to the cold compressing portion 11. The heat conducting portion 40 is in contact with the cold compressing portion 11, to absorb heat of the cold compressing portion 11. The cool driving portion 50 is fixed on one side of the hair removal device 30 away from the cold compressing portion 11, and is configured for driving external cooling medium to flow through a part of the heat conducting portion 40 away from the cold compressing portion 11, so that the heat of the heat conducting portion 40 can be taken away by the external cooling medium.

The head portion 10 includes the cold compressing portion 11 and the illuminating portion 12 arranged around the cold compressing portion 11. The illuminating portion 12 is configured to emit light for caring the skin of the user. The hair removal device 30 is directly opposite to the cold compressing portion 11, and is configured to emit light to the cold compressing portion 11, so that the cold compressing portion 11 can emit light to irradiate hair follicles of the skin of the user, and use the light to penetrate the skin to irradiate the hair follicles for hair removal, thus, the depilator 100 has both functions of hair removal and skin care.

It can be understood that the depilator 100 uses the cold compressing portion 11 to contact the skin of the user, and the cold compressing portion 11 emits light, and the light penetrates the skin to irradiate the hair follicles for hair removal. The cold compressing portion 11 can quickly cool down to reduce a burning sensation caused by the light and ensure comfort. The illuminating portion 12 emits light to the skin of the user, and the light is configured to care the skin of the user, so that the depilator 100 has multiple functions.

In this embodiment, the head portion 10 is located at one end of the hand-held portion 20. The hand-held portion 20 is columnar, so that the user can hold the hand-held portion 20. The cold compressing portion 11 is located in the head portion 10 and includes a cold compressing surface 111 exposing the head portion 10. The illuminating portion 12 is completely located in the head portion 10 and emits light from a periphery of the cold compressing surface 111. The light may be visible light such as red light, green light, yellow light, or so on. The hair removal device 30 is located at a part of the hand-held portion 20 adjacent to the head portion 10. The hair removal device 30 is located on one side of the cold compressing portion 11 away from the cold compressing surface 111. The hair removal device 30 emits light toward the cold compressing portion 11, and the light passes through the cold compressing portion 11 and irradiates the hair follicles of the skin of the user, to achieve hair removal. A part of the heat conducting portion 40 is in contact with the cold compressing portion 11, to absorb the heat of the cold compressing portion 11, and the other part of the heat conducting portion 40 is far away from the cold compressing portion 11, to conduct heat away, and to realize heat conduction to the cold compressing portion 11.

In this embodiment, the cool driving portion 50 drives the external cooling medium to flow into the depilator 100, so that the external cooling medium can take away the heat of the hair removal device 30 and the heat conducting portion 40. The cool driving portion 50 drives the external cooling medium to flow through a power device, so that the external cooling medium can effectively take away the heat in the depilator 100.

Optionally, the cool driving portion 50 is a fan. The external cooling medium is air. For example, the cool driving portion 50 is a centrifugal fan, an axial fan, a mixed flow fan, and a cross flow fan. Preferably, the cool driving portion 50 is a centrifugal fan, so that the external air enters an impeller of the fan axially and flows mainly in a radial direction.

Furthermore, the illuminating portion 12 includes a light source substrate 121 arranged around the cold compressing portion 11 and a plurality of lamp beads 122 arranged on the light source substrate 121. The plurality of lamp beads 122 are arranged equidistantly around the cold compressing portion 11 in a circumferential direction.

In this embodiment, the light source substrate 121 defines a through hole 123, and the cold compressing portion 11 passes through the through hole 123, to achieve the light source substrate 121 to be arranged around the peripheral side of the cold compressing portion 11. One surface of the light source substrate 121 where the lamp beads 122 are mounted is parallel to the cold compressing surface 111. The lamp beads 122 are located on one surface of the light source substrate 121 close to the cold compressing surface 111. The light source substrate 121 drives the plurality of lamp beads 122 to emit light. The lamp bead 122 is an LED (Light Emitting Diode, light-emitting diode). The lamp bead 122 can convert the emitted light energy into intracellular energy, accelerate a cycle of a cell growth process, stimulate fiber cells to produce collagen, and will not cause any damage or discomfort to the skin. The plurality of lamp beads 122 are equidistantly arranged the cold compressing portion 11 in a circumferential direction, so that the head portion 10 emits light uniformly on the peripheral side of the cold compressing portion 11, and improves a skin care effect to the user. The plurality of lamp beads 122 can all emit light of the same color, or light of multiple different colors.

It can be understood that the light emitted by the lamp beads 122 acts on the skin of the user via the head portion 10 and cares the skin of the user. By arranging the plurality of lamp beads 122 on the depilator 100, the depilator 100 has a function of hair removal and also has a function of skin beauty, and the lamp beads 122 can repair the skin damage caused by the hair removal device 30 during hair removal.

Specifically, the depilator 100 can set a switch to adjust the lamp beads 122 to emit light of different colors. The plurality of lamp beads 122 can emit any one or a combination of multiple colors such as red light, blue light, yellow light and so on. The plurality of lamp beads 122 in the illuminating portion 12 include at least one red lamp emitting red light, at least one blue lamp emitting blue light, and at least one yellow lamp emitting yellow light. The lamp beads 122 emitting light of different colors are arranged alternately in sequence .

Optionally, the lamp bead 122 can emit red light, and the red light exits through the head portion 10 and irradiates the skin of the user. The red light emitted by the lamp bead 122 can improve the activity of skin cells and promote the metabolism of skin cells. The red light can also improve fine wrinkles and prevent skin from sagging and wrinkle removal. Specifically, the wavelength of the red light is 620nm-660nm. The wavelength of the red light may also specifically be 630nm, 632nm, 635nm, 637nm, 640nm, 643nm, 647nm or 650nm. Preferably, the wavelength of the red light is 630nm. Red light waves in this wavelength range can stimulate the blood flow of the skin, promote the growth of collagen, stimulate cell activity and promote renewal growth, and can achieve the effects of whitening and rejuvenating skin and repairing skin damage.

Optionally, the lamp bead 122 can emit blue light, and the blue light is emitted through the head portion 10 and irradiated on the skin of the user. The blue light emitted by lamp bead 122 can kill bacteria on the skin and inhibit the growth of bacteria. Blue light can also improve sebum, reduce inflammation, acne, and shrink pores. Specifically, the blue light has a wavelength of 450nm-490nm. Specifically, the wavelength of the blue light may specifically be 450nm, 460nm, 467nm, 480nm, 485nm or 490nm. Preferably, the wavelength of the blue light is 470nm. The blue light waves in this wavelength range have a calming and anti-inflammatory effect and can inhibit the growth of bacteria.

Optionally, the lamp bead 122 can emit yellow light, and the yellow light is emitted through the head portion 10, and irradiated on the skin of the user. The yellow light emitted by the lamp bead 122 can improve dark yellow skin and freckle removal effects. Specifically, the wavelength of the yellow light is 567nm-607nm, and the wavelength of the yellow light may also be 577nm, 580nm, 581nm, 585nm, 588nm, 590nm, 595nm or 597nm. Preferably, the wavelength of the yellow light is 590nm. The yellow light wave in this wavelength range has an effect of improving the exchange rate of cell nutrients, and can also replenish energy to the skin of the human body and improve the roughness of the skin.

With the depilator 100 of this design, while the cold compressing portion 11 is in contact with the skin to remove hair, the lamp beads 122 can further play a role of skin care and skin beauty. The integrated design of the lamp beads 122 and the cold compressing portion 11 can combine skin care and hair removal together, the functions are rich and the user experience is improved.

Furthermore, the head portion 10 further includes a light cover 13. The light cover 13 covers the plurality of lamp beads 122, and defines a mounting hole 131 for clearance fit with the cold compressing portion 11. One surface of the cold compressing portion 11 away from the hair removal device 30 is flush with one surface of the light cover 13 away from the lamp beads 122.

In this embodiment, the light cover 13 includes an ending plate 132 and a connecting plate 133 extending from a periphery of the ending plate 132. The inner sides of the ending plate 132 and the connecting plate 133 form a receiving cavity. The lamp beads 122 and the light source substrate 121 are received in the receiving cavity, so that the light cover 13 protects the lamp beads 122 and the light source substrate 121. The light emitted by the lamp beads 122 passes through the ending plate 132 and is emitted to the skin of the user. The light cover 13 can be made of transparent or semi-transparent materials. One surface of the ending plate 132 away from the lamp beads 122 is flush with the cold compressing surface 111, so that one surface of the head portion 10 that contacts the skin of the user is smooth, and the comfort is increased. The connecting plate 133 is configured to detachably connect with the hand-held portion 20 to facilitate the disassembly and maintenance of the lamp beads 122 and the light source substrate 121.

Furthermore, the hand-held portion 20 includes a housing 21. One end of the housing 21 is abutted with the light cover 13. The cold compressing portion 11, the hair removal device 30, the cool driving portion 50 and the heat conducting portion 40 are all fixed in the housing 21.

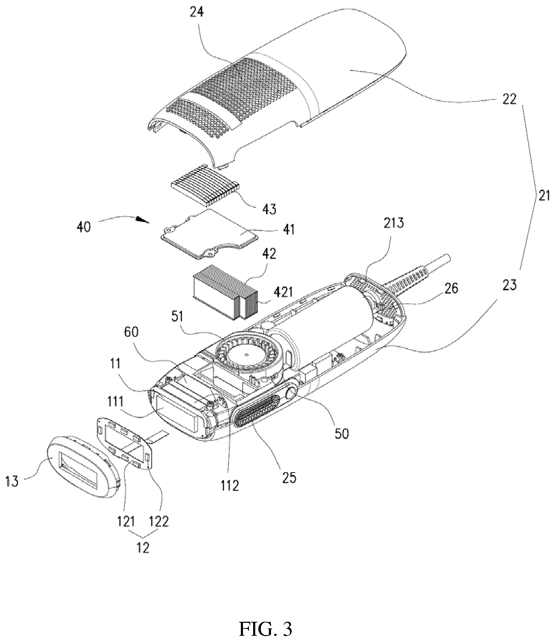

In this embodiment, the housing 21 includes an upper housing 22 and a lower housing 23. The upper housing 22 and the lower housing 23 can be assembled by screwing or clamping, or can be integrally processed. Optionally, the upper housing 22 and the lower housing 23 are assembled by snap-fitting, so as to facilitate disassembly and maintenance of the upper housing 22 and the lower housing 23. The housing 21 defines a receiving space 211. The hair removal device 30, the cool driving portion 50 and the heat conducting portion 40 are all fixed in the receiving space 211. The cool driving portion 50 drives the external cooling medium to flow into the housing 21, so that both the hair removal device 30 and the heat conducting portion 40 can contact the external cooling medium, and finally use the external cooling medium to absorb the heat of the hair removal device 30 and the heat conducting portion 40, and discharge the heat from the housing 21. The housing 21 defines a head port 212 between the upper housing 22 and the lower housing 23, and the connecting plate 133 is inserted into the head port 212. The connecting plate 133 is fixedly coupled to the upper housing 22 and the lower housing 23, and an outer surface of the connecting plate 133 is flush with an outer surface of the upper housing 22 and an outer surface of the lower housing 23. The housing 21 also includes a tail cover 213 opposite to the head port 212 (see FIG. 3 ). The tail cover 213 is fixedly connected between the upper housing 22 and the lower housing 23, and is located away from the head portion 10. The tail cover 213 is configured for a cable passing through and entering into the receiving space 211, so as to facilitate the depilator 100 to obtain power or obtain control signals.

Furthermore, referring to FIGS. 3, 4 and 5, the housing 21 defines a first guide opening 24 and a second guide opening 25. The first guide opening 24 is adjacent to the cool driving portion 50, and the second guide opening 25 is adjacent to the hair removal device 30. A cooling channel is formed between the first guide opening 24 and the second guide opening 25. The cool driving portion 50 is configured to drive the external cooling medium in the cooling channel to flow.

In this embodiment, the first guide opening 24 is defined in the upper housing 22. The first guide opening 24 interconnects with the receiving space 211 in the housing 21. The second guide opening 25 is arranged at a joint of the upper housing 22 and the lower housing 23. The cool driving portion 50 is located in an area between the first guide opening 24 and the second guide opening 25. The cool driving portion 50 defines a first driving opening 51 abutted with the first guide opening 24 and a second driving opening 52 facing the hair removal device 30 and the cold compressing portion 11. The first driving opening 51 is configured to input or output the external cooling medium from the first guide opening 24, and the second driving opening 52 is configured to blow or suck away the cooling medium to the hair removal device 30 and the cold compressing portion 11, thereby enabling the cooling medium around the hair removal device 30 and the cold compressing portion 11 to flow, so that the cooling medium can absorb the heat of the hair removal device 30 and the cold compressing portion 11 and then discharge the heat. Specifically, the cool driving portion 50 includes a shell 53 and a fan blade fixed in the shell 53. The first driving opening 51 and the second driving opening 52 are opened in the shell 53. The shell 53 is fixed in the housing 21. The shell 53 is located on one side of the hair removal device 30 away from the cold compressing portion 11. The first driving opening 51 is opened on a top portion of the shell 53 adjacent to the upper housing 22. The second driving opening 52 is opened at one end of the shell 53 facing the hair removal device 30 and the cold compressing portion 11. The first driving opening 51 may be a cooling medium input opening, that is, the cool driving portion 50 sucks in the external cooling medium from the first guide opening 24 through the first driving opening 51. The second driving opening 52 blows the external cooling medium toward the hair removal device 30 and the cold compressing portion 11, and finally the cooling medium that has absorbed heat is output from the second guide opening 25 to the outside of the housing 21. Of course, the first driving opening 51 may also be a cooling medium output opening, that is, the first driving opening 51 outputs the cooling medium to the first guide opening 24, and the second driving opening 52 removes the cooling medium around the hair removal device 30 and the cold compressing portion 11 away, so that the heat of the hair removal device 30 and the cold compressing portion 11 is taken away.

Furthermore, the depilator 100 further includes a cooling bracket 60 fixed in the housing 21. The cooling bracket 60 defines a first cooling channel 61 and a second cooling channel 62 separated from the first cooling channel 61. The hair removal device 30 is fixed in the first cooling channel 61. A part of the heat conducting portion 40 away from the cold compressing portion 11 is fixed in the second cooling channel 62. The end of the cool driving portion 50 is abutted with the first cooling channel 62. A peripheral side of the cool driving portion 50 is abutted with the first cooling channel 61 and the second cooling channel 62. The ends of the first cooling channel 61 and the second cooling channel 62 away from the cool driving portion 50 are abutted with the second guide opening 25.

In this embodiment, the cooling bracket 60 is fixedly coupled to the cool driving portion 50, and defines the first cooling channel 61 and the second cooling channel 62 that are abutted with the second driving opening 52. The first cooling channel 61 and the second cooling channel 62 are separated. The hair removal device 30 is fixed in the first cooling channel 61, so as to use the cooling medium in the first cooling channel 61 to take away heat. The cold compressing portion 11 is fixed to the cooling bracket 60 and is opposite to the hair removal device 30, to receive the light emitted by the hair removal device 30 and emit the light. The heat conducting portion 40 is fixed to the cooling bracket 60, one part of the heat conducting portion 40 is in contact with the cold compressing portion 11, and the other part of the heat conducting portion 40 is arranged in the second cooling channel 62, to absorb the heat of the cold compressing portion 11 and guide the heat of the cold compressing portion 11 to the second cooling channel 62, and take away the heat by using the cooling medium in the second cooling channel 62.

As the cooling bracket 60 defines the first cooling channel 61 and the second cooling channel 62, both of the first cooling channel 61 and the second cooling channel 62 are abutted with the second driving opening 52 of the cool driving portion 50, and are fixed in the first cooling channel 61 by using the hair removal device 30, one part of the heat conducting portion 40 is in contact with the cold compressing portion 11, and the other part of the heat conducting portion 40 is arranged in the second cooling channel 62, so that the heat dissipation of the heat conducting portion 40 is separated from the heat dissipation of the heat removal device 30, thereby effectively improving an interior overall cooling efficiency of the depilator 100.

In this embodiment, the cool driving portion 50 drives the external cooling medium to flow into the depilator 100 with dual cooling channels, so that the external cooling medium flows into the first cooling channel 61 and the second cooling channel 62 which facilitates the external cooling medium to take the heat of the hair removal device 30 and the heat conducting portion 40 away at the same time. Specifically, the outer side of the second driving opening 52 is snap-coupled to the cooling bracket 60, so that the second driving opening 52 is sealed and abutted with the first cooling channel 61 and the second cooling channel 62, thereby reducing a resistance of the external cooling medium into the first cooling channel 61 and the second cooling channel 62.

The cool driving portion 50 drives the external cooling medium to flow through a power device, so that the external cooling medium can effectively take away the heat in the depilator 100 with the dual cooling channels. The second driving opening 52 is abutted with the first cooling channel 61 and the second cooling channel 62 which facilitates the cool driving portion 50 to use the power device to drive the external cooling medium to cool down the hair removal device 30 and the heat conducting portion 40 respectively from the two cooling channels, optimizes an internal structure of the depilator 100 with the dual cooling channels, and increases an internal heat dissipation efficiency of the depilator 100 with the dual cooling channels.

In this embodiment, the cooling bracket 60 includes a bracket head 69, and the cold compressing portion 11 is fixed to the bracket head 69 so that the cold compressing portion 11 contacts the skin of the user. The illuminating portion 12 abuts against the bracket head 69, and one surface of the light source substrate 121 away from the lamp beads 122 abuts against the end surface of the bracket head 69, so that the illuminating portion 12 and the cooling bracket 60 are stable, and the light cover 13 is firmly coupled to the bracket head 69, to effectively cover the illuminating portion 12. The cooling bracket 60 is abutted with the second driving opening 52 at an opposite or adjacent side of the bracket head 69. The first cooling channel 61 and the second cooling channel 62 are abutted with the second driving opening 52 side by side, and the first cooling channel 61 and the second cooling channel 62 are separated by a partition.

In this embodiment, the hair removal device 30 emits light toward the cold compressing portion 11. The heat of the hair removal device 30 can be taken away by the external cooling medium of the first cooling channel 61. The external cooling medium of the first cooling channel 61 is directly obtained by the cool driving portion 50 sucking in the cooling medium of which external temperature is lowered, so as to effectively reduce the temperature of the hair removal device 30.

In this embodiment, the cold compressing portion 11 partially exposes the bracket head 69 so that the cold compressing portion 11 contacts the skin of the user. The cold compressing portion 11 is configured to contact the skin of the user and emit light. The cold compressing portion 11 has a cold compressing surface 111, and the cold compressing surface 111 is parallel to an outer end surface of the bracket head 69. The cold compressing portion 11 also has a heat conducting surface 112 that exposes the bracket head 69 and is arranged on a peripheral side of the cold compressing portion 11. The heat conducting surface 112 is attached to a part of the heat conducting portion 40, so that the heat of the cold compressing portion 11 is conducted from the heat conducting surface 112 to the heat conducting portion 40, thus, the cold compressing portion 11 achieves a cooling effect, and the cooling rate is accelerated.

Optionally, the cold compressing surface 111 is a flat surface, and the heat conducting surface 112 is a flat surface.

Optionally, the cold compressing surface 111 is an end surface of the cold compressing portion 11. The heat conducting surface 112 is a side surface of the cold compressing portion 11.

In this embodiment, a part of the heat conducting portion 40 is fixed to the bracket head 69 and is in contact with the heat conducting surface 112 of the cold compressing portion 11, and the other part of the heat conducting portion 40 extends from a part of the cooling bracket 60 away from the bracket head 69 into the second cooling channel 62, such that the external cooling medium in the second cooling channel 62 takes away the heat of the cold compressing portion 11, to reduce the temperature of the cold compressing portion 11 and reduce a burning sensation to the skin of the user caused by the light emitted by the cold compressing portion 11.

Furthermore, the heat conducting portion 40 includes a heat conducting plate 41 attached to the cold compressing portion 11 and a first heat sink assembly 42 and a second heat sink assembly 43 which are attached to the heat conducting plate 41. The heat conducting plate 41 is fixed on the cooling bracket 60. One end of the heat conducting plate 41 away from the cold compressing portion 11 covers the second cooling channel 62. The first heat sink assembly 42 is received in the second cooling channel 62 and contacts with a part of the heat conducting plate 41 covering the second cooling channel 62. The second heat sink assembly 43 is fixed on one side of the heat conducting plate 41 away from the first heat sink assembly 42, and the second heat sink assembly 43 is adjacent to the first guide opening 24, to increase an area of the heat conducting portion 40 contacting the external cooling medium, and increase a heat exchange rate between the heat conducting portion 40 and the external cooling medium, thereby increasing a cooling rate of the cold compressing portion 11 to the heat conducting portion 40.

In this embodiment, the heat conducting plate 41 is a flat plate. The heat conducting plate 41 adopts a structure in which two plates are laminated and sealed, and a heat conducting net is arranged between the two plates. A heat conducting medium is also arranged between the two plates of the heat conducting plate 41. The heat conducting net is fully contacted with the heat conducting medium, so that the contact area of the heat conducting medium contacting the two plates and the heat conducting net increases, and the heat conducting medium can quickly absorb the heat that the heat conductive plate 41 absorbs from the cold compressing portion 11, and the heat conducting medium can quickly conducts the heat to the first heat sink assembly 42, so that the heat can be dissipated by using the first heat sink assembly 42. The heat conducting plate 41 is a short heat conducting plate 41, that is, the distance between the place where the heat conducting plate 41 contacts the cold compressing portion 11 and the place where it contacts the first heat sink assembly 42 is short, so that the heat conducting plate 41 can conduct heat more quickly to the first heat sink assembly 42, and the first heat sink assembly 42 can quickly take away heat by using the cooling medium in the second cooling channel 62, so as to quickly cool down the cold compressing portion 11 and improve a cooling rate of the depilator 100.

Optionally, the two plates of the heat conducting plate 41 are both copper plates. The heat conducting net is a copper net with a capillary structure, so that the contact area of the heat conducting medium and the two plates is increased and more even, and the heat conduction efficiency of the heat conducting plate 41 is improved.

Optionally, a vacuum sealed cavity is provided between the two plates of the heat conducting plate 41, and the heat conducting medium and the heat conducting net are received in the sealed cavity.

Optionally, the heat conducting medium of the heat conducting plate 41 is cooling liquid, preferably water.

Optionally, the edges of the two plates of the heat conducting plate 41 are sealed and pressed together, so that the heat conducting plate 41 has a stable structure and prevents the heat from entering the heat conducting plate 41.

In this embodiment, the first heat sink assembly 42 includes a plurality of side-by-side heat sinks 421 attached to the heat conducting plate 41. The heat sinks 421 are configured to contact the heat conducting plate 41, to increase the contact area between the first heat sink assembly 42 and the heat conducting plate 41, and the heat conducting plate 41 can be configured to seal the second cooling channel 62, to avoid the leakage of the external cooling medium in the second cooling channel 62. The heat sinks 421 can quickly absorb the heat of the heat conducting plate 41. There is a cooling medium flow space between the plurality of heat sinks 421, and a cooling medium flow direction between the plurality of heat sinks 421 is consistent with an extending direction of the second cooling channel 62, so that the cooling medium in the second cooling channel 62 can quickly flow through the flow space between the plurality of heat sinks 421, such that the cooling medium in the second cooling channel 62 can quickly absorb the heat of the heat sinks 421 and take the heat away, thereby achieving effective heat dissipation of the heat conducting plate 41. The second heat sink assembly 43 and the first heat sink assembly 42 have substantially the same structure, which will not be repeated here.

Furthermore, the cooling bracket 60 includes a bottom shell 63 and a top shell 64 which is covered with the bottom shell 63. The heat conducting plate 41 is fixed on one side of the top shell 64 away from the bottom shell 63. A part of the cold compressing portion 11 is fixed between the bottom shell 63 and the top shell 64, and the other part of the cold compressing portion 11 passes through the top shell 64 to contact the heat conducting plate 41. The first cooling channel 61 is formed between the bottom shell 63 and the top shell 64. The second cooling channel 62 is formed between the bottom shell 63 and the heat conducting plate 41.

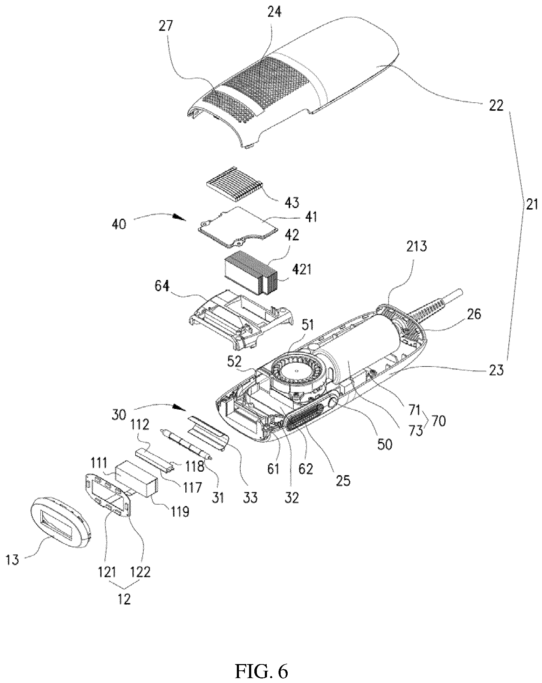

In this embodiment, the top shell 64 defines a gap 641 interconnecting with the second cooling channel 62. The heat conducting plate 41 seals and covers the gap 641, to achieve the heat conducting portion 40 to seal the gap 641, which facilitate a part of the heat conducting portion 40 to extend into the second cooling channel 62. The heat sinks 421 are completely received in the second cooling channel 62. A receiving groove 622 interconnecting with the first cooling channel 61 is also defined between the top shell 64 and the bottom shell 63. The cold compressing portion 11 is fixed in the receiving groove 622 so that the light from the hair removal device 30 can irradiate the cold compressing portion 11. The heat conducting surface 112 (see FIG. 6 ) extends out the receiving groove 622 from the top shell 64. The cold compressing surface 111 extends out the receiving groove 622 from the end surface of the bracket head 69, so that the cold compressing surface 111 is in contact with the skin of the user. The top shell 64 effectively supports the heat conducting plate 41, and the size of the heat conducting plate 41 is equivalent to the size of the top shell 64, making the internal structure of the depilator 100 more compact, which can effectively reduce the volume of the depilator 100 with the the dual cooling channels and is convenient to carry.

In this embodiment, the cooling bracket 60 defines a first transmitting opening 66 that interconnects with the first cooling channel 61 and the second cooling channel 62 and is away from the second driving opening 52. The first transmitting opening 66 is arranged between the top shell 64 and the bottom shell 63. The first transmitting opening 66 is disposed on a sidewall of the cooling bracket 60 adjacent to the cold compressing portion 11 and is away from the first driving opening 51.

Specifically, half of the first transmitting opening 66 is defined on the top shell 64 and the other half of the first transmitting opening 66 is defined on the bottom shell 63. The cooling bracket 60 defines the first transmitting opening 66 on a sidewall opposite to the cold compressing portion 11. The second driving opening 52 is abutted with the other sidewall of the cooling bracket 60. The cooling bracket 60 defines a second transmitting opening 65 on the other sidewall adjacent to the cold compressing portion 11. The second driving opening 52 of the shell 53 is abutted with the second transmitting opening 65. The first cooling channel 61 and the second cooling channel 62 are formed between the second transmitting opening 65 and the first transmitting opening 66. The cool driving portion 50 is located on one side of the cooling bracket 60 so that the second driving opening 52 blows the cooling medium to the second transmitting opening 65 and discharges the cooling medium from the first transmitting opening 66. The inner cooling medium of the cooling bracket 60 is blown in from one side of the cooling bracket 60 and blown out from the other side of the cooling bracket 60, so that the flow rate of the cooling medium in the first cooling channel 61 and the second cooling channel 62 increases, thereby increasing a heat dissipation efficiency.

In this embodiment, the cool driving portion 50, the cooling bracket 60, and the heat conducting portion 40 are all fixed in the receiving space 211. The second guide opening 25 is abutted with the first transmitting opening 66, such that the cooling medium entering into the cooling bracket 60 can be quickly discharged from the second guide opening 25, to prevent the cooling medium that has absorbed heat from flowing back into the cooling bracket 60. Of course, there may also be a certain distance between the second guide opening 25 and the first transmitting opening 66, so that the cooling medium discharged from the second guide opening 25 can be partially discharged from the first transmitting opening 66, and partially entering into the housing 21, so that the cooling medium can continue to cool down other components in the housing 21.

In this embodiment, the housing 21 protects the cool driving portion 50. The cool driving portion 50 directly sucks in the external cooling medium through the first guide opening 24, to reduce a resistance of the cool driving portion 50 driving the cooling medium to flow, and increase a flow rate of the cooling medium, and increase an internal heat dissipation rate of the depilator 100. Of course, there may also be a certain distance between the first driving opening 51 and the first guide opening 24, so that a part of the cooling medium entering from the first guide opening 24 can be quickly sucked into the cool driving portion 50, and is blown into the first cooling channel 61 and the second cooling channel 62, and the other part of the cooling medium can enter the housing 21 to cool down other components in the housing 21.

More specifically, the tail cover 213 defines a third guide opening 26, which can discharge the cooling medium in the housing 21, to facilitate the cooling medium which enters the housing 21 and is outside of the cooling bracket 60 to absorb the heat of other components in the housing 21, and discharge the heat from the third guide opening 26, so as to cool down the entire interior of the depilator 100 with the dual cooling channels.

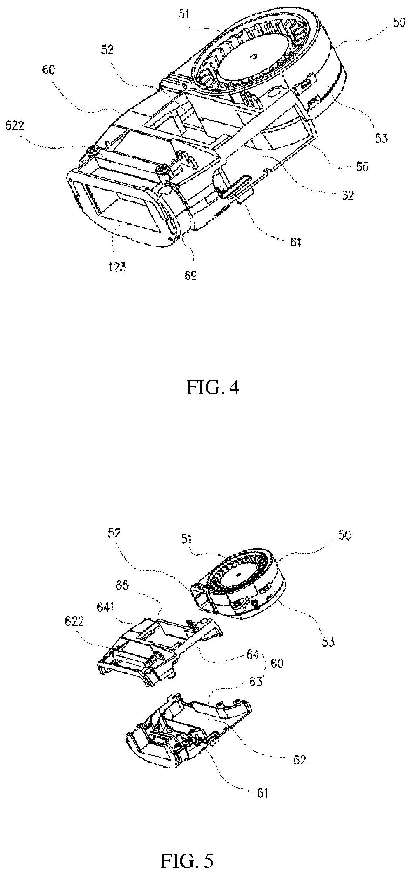

Furthermore, referring to FIGS. 4, 5 and 6 , the hair removal device 30 includes a lamp tube 31, a lamp tube holder 32, and a reflecting plate 33. The lamp holder 32 is fixed in the cooling bracket 60 and is close to the cold compressing portion 11. The lamp tube 31 and the reflecting plate 33 are fixed on the lamp tube holder 32. The reflecting plate 33 is located on one side of the lamp tube 31 away from the cold compressing portion 11. The reflecting plate 33 collects the light of the lamp tube 31 on the cold compressing portion 11.

In this embodiment, the lamp tube 31, the reflecting plate 33 and the lamp tube holder 32 are all located in the first cooling channel 61. The lamp tube 31, the reflecting plate 33 and the lamp tube holder 32 are all fixed in a part of the first cooling channel 61 adjacent to the cold compressing portion 11, to reduce a light path of the lamp tube 31, avoiding the heat of the emitted light conducting to more devices, and reducing the heat concentration in the first cooling channel 61. More specifically, the lamp tube holder 32 is fixed between the bottom shell 63 and the top shell 64, so that the lamp tube holder 32 and the cooling bracket 60 have a stable structure. The lamp tube 31, the reflecting plate 33 and the lamp tube holder 32 are adjacent to the first transmitting opening 65, so that the cooling medium which absorbs the heat of the lamp tube 31, the reflecting plate 33 and the lamp tube holder 32 can be discharged from the first transmitting opening 65 immediately, thereby effectively reducing the heat of the lamp tube 31, the reflecting plate 33 and the lamp tube holder 32. The reflecting plate 33 condenses and reflects the light of the lamp tube 31 to increase a light concentration of the cold compressing portion 11. The two ends of the reflecting plate 33 have openings, so that the cooling medium flows from one opening of the reflecting plate 33 to the other opening, and the external cooling medium contacts the peripheral side of the lamp tube 31 to form convection, which effectively cools the lamp tube 31. The two openings of the reflection plate 33 are respectively adjacent to the first transmitting opening 66 and the second transmitting opening 65 so that the cooling medium in the first cooling channel 61 quickly flows through an inner side of the reflection plate 33 to improve a heat dissipation efficiency of the lamp tube 31.

Furthermore, the depilator 100 with the dual cooling channels further includes a control device 70. The control device 70 includes a circuit board 71, a processor, and a capacitor 73. The processor is located on the circuit board 71. The capacitor 73 is electrically coupled to the circuit board 71. The circuit board 71 is electrically coupled to the cold compressing portion 11 and the hair removal device 30.

] In this embodiment, the hair removal device 30 is electrically coupled to the control device 70. The control device 70 can provide power to the hair removal device 30 and control an operation of the hair removal device 30, so that the depilator 100 can be used without plugging in an external circuit, or it can be used to directly connect an external power source, such as a dry capacitor or an energy storage capacitor, which is convenient for storage and carrying, and can be used outside. The control device 70 can also be coupled to an external circuit for charging, and can control the start and stop, power regulation, thermal protection, etc of the depilator 100 with dual cooling channels.

It is understandable that the heat inside the fuselage of the traditional depilator 100 is large at present, which is likely to cause short-circuit, burnout, explosion and other dangers to the components inside the fuselage of the depilator 100. However, the heat conducting plate 41 of the present disclosure uses the cooling medium in the second cooling channel 62 to effectively cool down the cold compressing portion 11. The hair removal device 30 uses the cooling medium in the first cooling channel 61 to effectively dissipate heat, so that the cold compressing portion 11 can effectively cold compress the skin of the user with high comfort and no skin damage, and the temperature of the hair removal device 30 is effectively reduced. The cooling medium can also be configured to cool down other internal components of the depilator 100 with dual cooling channels, so that the entire body of the depilator 100 with dual cooling channels is cooled, preventing the internal temperature of the depilator 100 with dual cooling channels from being too high and causing harm. Specifically, the control device 70 of the depilator 100 with dual cooling channels of the present disclosure generates heat when it is working. The cool driving portion 50 can also drive the external cooling medium to flow into the housing 21 to contact the control device 70, the heat of the control device 70 can be taken away by using external cooling medium, to realize the rapid cooling of the internal components of the depilator 100 with dual cooling channels and the control device 70. Excessive heat in the receiving space 211 of the depilator 100 with dual cooling channels can be avoided. The hair removal device 30, the control device 70 and other devices can be prevented from being short-circuited, burned, and exploded.

In this embodiment, the control device 70 is arranged inside the housing 21, and is optionally arranged on a part of the cooling bracket 60 away from the cold compressing portion 11. The control device 70 is arranged on one side of the hair removal device 30 away from the cold compressing portion 11, that is, one side of the hair removal device 30 away from the human skin. Through such a design, the contact range of the external cooling medium allowed to be contacted in the receiving space 211 is expanded to achieve an effect of overall internal cooling of the housing 21.

In this embodiment, the control device 70 includes the circuit board 71 fixed on the inner side of the lower housing 23, the processor fixed on the circuit board 71, and the capacitor 73 fixed on the inner side of the lower housing 23. The circuit board 71 is fixed in the part of the housing 21 close to the cooling bracket 60. The processor 72 processes the electrical signals of the hair removal device 30 and the illuminating portion 12, so as to control the hair removal device 30 to operate. The capacitor 73 provides power to the illuminating portion 12, the hair removal device 30, the cool driving portion 50, the circuit board 71, and the processor.

Furthermore, the cold compressing portion 11 includes a cold compressing member 119 and a cooling member 118 for cooling the cold compressing member 119. The cold compressing member 119 is configured to contact the skin of the user and perform cold compresses. The cooling element 118 includes a cooling surface 117 adhering to the cold compressing member 119. The cooling surface 117 is disposed opposite to the heat conducting surface 112. The heat conducting plate 41 is attached to the heat conducting surface 112 to absorb heat from the heat conducting surface 112.

In this embodiment, the lamp tube 31, the cooling member 118, and the light source substrate 121 are all electrically coupled to the circuit board 71. The cold compressing member 119 is clamped on the cooling bracket 60, so that the cooling member 118 is firmly attached to between the cold compressing member 119 and the cooling bracket 60, to facilitate the heat conducting plate 41 to contact with the cooling member 118. The cooling member 118 is closely attached to the cold compressing member 119, to cool the cold compressing member 119. A filter is also provided between the cold compressing member 119 and the lamp tube 31. The filter is configured to filter out some harmful light emitted from the lamp tube 31, so that the light emitted by the lamp tube 31 can safely and effectively remove hair on the skin of the user. The heat of the lamp tube 31 is conducted to the cold compressing member 119, and the reflecting plate 33 is arranged on the side of the lamp 31 away from the cold compressing member 119, so that the light emitted by the lamp 31 is concentrated on the cold compressing member 119.

Preferably, the cold compressing member 119 is made of crystal materials, and specifically can be sapphire, K9 glass, crystal glass, and any materials that satisfies light-transmitting crystals, and optionally sapphire materials.

It can be understood that since the cold compressing member 119 is made of sapphires, the cold compressing member 119 can also be used as a light outlet. When the lamp tube 31 emits light, the sapphire has strong thermal conductivity, so that the cooling element 118 and the cold compressing member 119 can produce heat exchange efficiently, so as to achieve the best cooling effect. Optionally, the cold compressing member 119 may be a circular plate or a rectangular plate, which is not limited here. The side of the cold compressing member 119 away from the lamp tube 31 is in contact with the human body, and the contact surface may be a curved surface or a flat surface, preferably a flat surface.

In this embodiment, the lamp tube 31 may be an IPL lamp tube, which is located on one side of the cold compressing member 119 away from the human body. The light emitted by the lamp tube 31 is emitted to the skin of the user through the cold compressing member 119. The color of the light emitted by the lamp tube 31 is not limited, and can be colored light, composite light, etc. The specific wavelength and frequency are determined according to the usage.

In this embodiment, the depilator 100 with dual cooling channels includes a skin detection part. The skin detection part can be integrated in the cold compressing member 119. The skin detection part uses a capacitor touch detection principle to electrically connect the circuit board 71. When the cold compressing member 119 touches the skin, the internal preset capacitor detection device simultaneously detects whether the depilator 100 with dual cooling channels actually touches the skin, reducing users safety problems caused by misoperations.

In this embodiment, the shape of the reflecting plate 33 is not limited, and only the light of the lamp tube 31 is concentrated in the direction of the cold compressing member 119. Optionally, the reflecting plate 33 is U-shaped, and the opening of the reflecting plate 33 set toward the cold compressing member 119. The lamp tube 31 is located at a center of the U-shaped opening of the reflecting plate 33. In addition to concentrating light, it can also prevent the lamp tube 31 from radiating heat to other places during operation.

In this embodiment, the cooling member 118 can optionally be a cooling element having a semiconductor cooling method.

It can be understood that due to the special principle of the depilator 100 with dual cooling channels adopts light hair removal technology, a lot of heat will be generated when the light is emitted. At the same time, when the cold compressing member 119 touches the skin, in order to prevent the light from causing the user to bum for pain, a cooling structure needs to be provided in the body of the depilator 100 with dual cooling channels. No matter how the temperature is lowered, heat will be generated during the heat exchange. The cooling member 118 is close to the heat conducting plate 41, making the cold compressing member 119 fast cooling. In addition to the heat generated by the cooling member 118, the plurality of heating devices are also arranged in the receiving space 211, and the external cooling medium in the housing 21 can effectively cool the plurality of heating devices.

It can be understood that the external cooling medium is sucked in from the first guide opening 24, enters the first driving opening 51, and then passes through the second driving opening 52 to the cooling bracket 60, and then passes through the hair removal device 30 and the first heat sink assembly 42. The heat of the lamp tube 31 is decreased, and the heat of the heat conducting plate 41 and the cold compressing portion 11 are decreased. Part of the cooling medium enters the housing 21, flows through the circuit board 71, the processor and the capacitor 73, and is finally discharged from the third guide opening 26, so that the area where the external cooling medium passes through is cooled.

Furthermore, the housing 21 further defines a fourth guide opening 27 adjacent to the first guide opening 24. The fourth guide opening 27 is directly opposite to the heat conducting portion 40. A cooling channel is formed between the fourth guide opening 27 and the first guide opening 24, so that the heat of the heat conducting portion 40 is taken away by the external cooling medium in the cooling channel. The fourth guide opening 27 and the first guide opening 24 are arranged on a same side of the housing 21. The fourth guide opening 27 and the first guide opening 24 are located at one side of the first guide opening 24 close to the head portion 10. The fourth guide opening 27 can quickly conduct the heat of the heat conducting portion 40 away, so as to achieve a rapid cooling of the heat conducting portion 40.

Based on the above-mentioned separation of the first cooling channel 61 and the second cooling channel 62 in the depilator 100 with dual cooling channels, in some embodiments, the heat dissipation method of the depilator 100 may not use fan air cooling, or other external cooling channels may be used. The cooling medium, such as water, coolant, etc., only needs the external medium to take away the heat of the cooling channel.

A contact end surface of the cold compressing portion 11 with the skin and an exposed end surface of the skin detection part are located on the same side, so that when the skin is irradiated and hair removed, the cold compressing member 119 is in contact with the irradiated area of the skin to cool the irradiated skin, reducing the burning sensation of the irradiated skin. The cold compressing member 119 can be close to zero temperature. The skin near a light exit infinitely close to the freezing point is truly achieved, which can reduce a burning sensation of the skin, and will not cause a skin damage by a short-term contact.

In some embodiments, a depilator 100 includes a luminous body, and the structural features described in all the above embodiments are used.

Those skilled in the art should also understand that if all or part of the components of the depilator 100 with dual cooling channels of the present disclosure are combined by means of fusion, physical connection, etc., such as the moving positions of the components in the hair removal device 30; or they are integrated or detachable design; and the replacement of the number of features, and the change of the feature shape that is not used as a function. Any combined component can form an equipment/device with a specific function. Using such the equipment/device to replace the corresponding component of the present disclosure also falls within the protection scope of the present disclosure.

Compared with the prior art, the depilator 100 with dual cooling channels of the present disclosure has the following advantages.
1. The head portion 10 includes the cold compressing portion 11 and the illuminating portion 12 arranged around the cold compressing portion 11. The illuminating portion 12 is configured to emit light for caring the skin of the user. The hair removal device 30 is in directly contact with the cold compressing portion 11, and is configured to emit light to the cold compressing portion 11, so that the cold compressing portion 11 can emit light to irradiate the hair follicles of the skin of the user. By using the light to penetrate the skin to irradiate the hair follicles for hair removal, the depilator 100 has both functions of hair removal and skin care.
2. The cooling bracket 60 defines the first cooling channel 61 and the second cooling channel 62. Both the first cooling channel 61 and the second cooling channel 62 are abutted with the second driving opening 52 of the cool driving portion 50. The hair removal device 30 is fixed in the first cooling channel 61. A part of the heat conducting portion 40 is in contact with the cold compressing portion 11, and the other part of the heat conducting portion 40 is arranged in the second cooling channel 62. Thus, the heat dissipation of the heat conducting portion 40 and the heat dissipation of the hair removal device 30 are achieved to be separated, thereby effectively improving an overall internal cooling efficiency of the decoder 100.
3. The depilator 100 with dual cooling channels uses a composite structure of the heat conducting plate 41. The heat conducting plate 41 uses two laminated plates, a heat conduction medium and a heat conduction net sealed between the two plates to increase a heat conduction effectively of the heat conducting plate 41. The heat conducting plate 41 absorbs the heat of the cold compressing portion 11, so that the heat of the cold compressing portion 11 is rapidly reduced. The heat conducting plate 41 is made of copper materials, which increases the heat conduction efficiency of the heat conducting plate 41, can effectively and quickly absorb heat, and effectively and quickly dissipate heat.
4. In the past, only a sealed cavity was configured to separately protect the cooling member 118 of the cold compressing portion 11 to ventilate it. The hair removal device 30 and the control device 70, which also generate heat, were not considered, especially when used for a long time, the heat of the hair removal device 30 and the lamp tube 31 will be very high, so the cooling effect is not complete. The depilator 100 with dual cooling channels of the present disclosure separately simultaneously cools the cold compressing portion 11 and the hair removal device 30, so that the overall cooling effect is greatly improved, and the overall temperature in the body of the depilator 100 with dual cooling channels is effectively reduced.
5. The external cooling medium that can be used by the technical means of the present disclosure can be not only the introduction of air for the fan, but also other cooling mediums, such as water pumps, water extraction or cooling liquids, etc.
6. The first guide opening 24 is arranged on one side of the head portion 10, so that the cooling medium can be discharged quickly, and the heat dissipation efficiency of the cold compressing portion 11 and the hair removal device 30 is improved. The second guide opening 25 is arranged at a part of the housing 21 adjacent to the hair removal device 30 and the cold compressing portion 10, and facilitates the simultaneous removal of heat from the hair removal device 30 and the heat conducting portion 40, thereby increasing the heat dissipation efficiency. The third guide opening 26 is arranged at a tail end of the depilator 100 with dual cooling channels away from the cold compressing portion 11, so that the cooling medium flows in the depilator 100 with dual cooling channels and is discharged after a longer distance, and the heat flowing through the control device 70 is discharged, which can reduce the heat of the control device 70.
7. The cold compressing member 119 is exposed from the head portion 10 of the housing 21 and actually contacts the human skin, so that the user can feel a cold compress effect of the cold compressing member 119 during a cold compressing process by using the depilator 100 with dual cooling channels, thereby the user's strong pain sensation due to the heat of the depilator 100 with dual cooling channels is reduced.
8. The cold compressing member 119 is made of crystal materials, which can be made of sapphire. Sapphire has strong thermal conductivity, which can reduce the heat of the light emitted by the depilator 100 with dual cooling channels while maintaining the light transmittance.
9. The cooling element 118 can be a semiconductor cooling chip, which can effectively improve the cooling effect of the depilator 100 with dual cooling channels.
10. The depilator 100 with dual cooling channels is equipped with the skin detection part. When the cold compressing portion 11 is in contact with the skin, the skin detection part will detect whether the machine actually touches the skin, which can reduce the safety problems caused by the user due to misoperation, and improve the working efficiency of the depilator 100 with dual cooling channels.

The above are the implementation modes of the embodiments of the present disclosure. It should be pointed out that for those of ordinary skill in the art, several improvements and modifications can be made without departing from the principle of the embodiments of the present disclosure, and these improvements and modifications are also treated as the scope of protection of the present disclosure as defined in the claims.

## Claims

1. A depilator (100), wherein, the depilator comprises a head portion (10) and a hand-held portion (20) coupled to the head portion; the head portion comprises a cold compressing portion (11) and an illuminating portion 12 )arranged around the cold compressing portion; the illuminating portion is configured to emit light to care a skin of a user; the hand-held portion comprises a hair removal device (30), a heat conducting portion (40), and a cool driving portion (50); the hair removal device is directly opposite to the cold compressing portion, configured to emit light to the cold compressing portion; the heat conducting portion is in contact with the cold compressing portion, to absorb heat of the cold compressing portion; the cool driving portion is fixed on a side of the hair removal device away from the cold compressing portion, and configured to drive external cooling medium to flow through a part of the heat conducting portion away from the cold compressing portion, so that the heat of the heat conducting portion is taken away by the external cooling medium,
wherein the depilator (100) is **characterized by** further comprising a cooling bracket (60),
wherein the cooling bracket (60) is fixedly coupled to the cool driving portion (50) and defines a first cooling channel (61) and a second cooling channel (62), which is separated from the first cooling channel (61);
the hair removal device (30) is fixed in the first cooling channel (61); and
a part of the heat conducting portion (40) away from the cold compressing portion (11) is fixed in the second cooling channel (62).

2. The depilator according to claim 1, wherein the illuminating portion comprises a light source substrate (121) arranged around the cold compressing portion and a plurality of lamp beads (122) arranged on the light source substrate; the plurality of lamp beads are equidistantly arranged around the cold compressing portion in a circumferential direction.

3. The depilator according to claim 2, wherein the head portion further comprises a light cover (13); the light cover covers the plurality of lamp beads, and defines a mounting hole (131) for clearance fit with the cold compressing portion; one surface of the cold compressing portion away from the hair removal device is flush with one surface of the light cover away from the lamp beads.

4. The depilator according to claim 3, wherein the light cover is a transparent or semi-transparent cover.

5. The depilator according to claim 3, wherein the hand-held portion comprises a housing (21); one end of the housing is abutted with the light cover; the cold compressing portion, the hair removal device, the cool driving portion and the heat conducting portion are both fixed in the housing.

6. The depilator according to claim 5, wherein the housing defines a first guide opening (24) and a second guide opening (25); the first guide opening is adjacent to the cool driving portion; the second guide opening is adjacent to the hair removal device; a cooling channel is formed between the first guide opening and the second guide opening; the cool driving portion is configured to drive the external cooling medium to flow in the cooling channel.

7. The depilator according to claim 6, wherein the cooling bracket (60) is fixed in the housing; wherein the first cooling channel and the second cooling channel are parts of the cool channel; one end of the cool driving portion is abutted with the first guide opening; a peripheral side of the cool driving portion is abutted with the first cooling channel and the second cooling channel; ends of the first cooling channel and the second cooling channel away from the cool driving portion are abutted with the second guide opening.

8. The depilator according to claim 7, wherein the cooling bracket comprises a bottom shell (63) and a top shell (64) covering the bottom shell; the heat conducting portion is fixed to one side of the top shell away from the bottom shell; a part of the cold compressing portion is fixed to between the bottom shell and the top shell, and the other part of the cold compressing portion passes through the top shell to contact the heat conducting portion; the first cooling channel and the second cooling channel are formed between the bottom shell and the top shell.

9. The depilator according to claim 7, wherein the heat conducting portion comprises a heat conducting plate (41) attached to the cold compressing portion and a heat sink assembly (42) attached to the heat conducting plate; the heat conducting plate is fixed on the cooling bracket, one end of the heat conducting plate away from the cold compressing portion seals and covers the second cooling channel; the heat sink assembly is accommodated in the second cooling channel and contacts with a part of the heat conducting plate covering the second cooling channel.

10. The depilator according to claim 7, wherein the depilator further comprises a control device (70) fixed in the housing; the control device is electrically coupled to the hair removal device and the cold compressing portion, and is located one side of the hair removal device away from the cold compressing portion; one end of the housing away from the head portion defines a third guide opening (26); the cooling channel is further formed between the third guide opening and the first guide opening, so that the control device is located on the cooling channel and the heat of the control device is taken away by the external cooling medium.

11. The depilator according to claim 10, wherein the control device comprises a circuit board (71), a processor and a capacitor (73); the processor is arranged on the circuit board; the capacitor is electrically coupled to the circuit board; the circuit board is electrically coupled to the cold compressing portion and the hair removal device.

12. The depilator according to claim 10, wherein the housing further defines a fourth guide opening (27) adjacent to the first guide opening; the fourth guide opening is directly opposite to the heat conducting portion; the cooling channel is further formed between the fourth guide opening and the first guide opening, so that the heat of the heat conducting portion is taken away by the external cooling medium in the cooling channel.

13. The depilator according to any one of claims 1 to 12, wherein the cold compressing portion comprises a cold compressing member (119) and a cooling member (118) that cools the cold compressing member; the cold compressing member is configured to contact the skin of the user to perform cold compress; the heat conducting portion contacts the cooling member to absorb heat of the cooling member; and, the illuminating portion surrounds the cold compressing member.

14. The depilator according to any one of claims 7 to 12, wherein the hair removal device comprises a lamp tube (31), a lamp tube holder (32) and a reflecting plate (33); the lamp holder is fixed in the cooling bracket, and close to the cold compressing portion; the lamp tube and the reflecting plate are fixed on the lamp tube holder; the reflecting plate is located on one side of the lamp tube away from the cold compressing portion; the reflecting plate concentrates light of the lamp tube on the cold compressing portion.

## Patentansprüche

1. Ein Depilator (100), wobei der Depilator einen Kopfteil (10) und einen handgehaltenen Teil (20) umfasst, der mit dem Kopfteil gekoppelt ist; der Kopfteil umfasst einen Kältekompressionsteil (11) und einen Beleuchtungsteil (12), der um den Kältekompressionsteil angeordnet ist;
der Beleuchtungsteil ist dazu konfiguriert, Licht auszustrahlen, um die Haut eines Benutzers zu pflegen; der handgehaltene Teil umfasst ein Haarentfernungsgerät (30), einen Wärmeleitteil (40) und einen Kühlungstreiber (50); das Haarentfernungsgerät ist direkt gegenüber dem Kältekompressionsteil angeordnet, konfiguriert, um Licht auf den Kältekompressionsteil zu emittieren; der Wärmeleitteil ist in Kontakt mit dem Kältekompressionsteil, um dessen Wärme zu absorbieren; der Kühlungstreiber ist an einer Seite des Haarentfernungsgeräts angebracht, die vom Kältekompressionsteil weg zeigt, und ist konfiguriert, ein externes Kühlmedium durch einen Teil des Wärmeleitteils, der vom Kältekompressionsteil entfernt ist, fließen zu lassen, sodass die Wärme des Wärmeleitteils durch das externe Kühlmedium abgeführt wird,
wobei der Depilator (100) **dadurch gekennzeichnet ist, dass** er weiterhin einen Kühlungshalter (60) umfasst,
wobei der Kühlungshalter (60) fest mit dem Kühlungstreiber (50) verbunden ist und einen ersten Kühlkanal (61) und einen zweiten Kühlkanal (62) definiert, der vom ersten Kühlkanal (61) getrennt ist;
das Haarentfernungsgerät (30) ist im ersten Kühlkanal (61) befestigt; und
ein Teil des Wärmeleitteils (40), der vom Kältekompressionsteil (11) entfernt ist, ist im zweiten Kühlkanal (62) befestigt.

2. Der Depilator nach Anspruch 1, wobei der Beleuchtungsteil ein Lichtquellensubstrat (121) umfasst, das um den Kältekompressionsteil angeordnet ist, und eine Vielzahl von Lampenperlen (122), die auf dem Lichtquellensubstrat angeordnet sind; die Vielzahl von Lampenperlen ist in einem gleichmäßigen Abstand um den Kältekompressionsteil in zirkulärer Richtung angeordnet.

3. Der Depilator nach Anspruch 2, wobei der Kopfteil weiterhin eine Lichtabdeckung (13) umfasst; die Lichtabdeckung bedeckt die Vielzahl von Lampenperlen und definiert ein Montageloch (131) für eine Passung mit Spiel mit dem Kältekompressionsteil; eine Oberfläche des Kältekompressionsteils, die vom Haarentfernungsgerät entfernt ist, ist bündig mit einer Oberfläche der Lichtabdeckung, die von den Lampenperlen entfernt ist.

4. Der Depilator nach Anspruch 3, wobei die Lichtabdeckung eine transparente oder halbtransparente Abdeckung ist.

5. Der Depilator nach Anspruch 3, wobei der handgehaltene Teil ein Gehäuse (21) umfasst; ein Ende des Gehäuses stößt an die Lichtabdeckung; der Kältekompressionsteil, das Haarentfernungsgerät, der Kühlungstreiber und der Wärmeleitteil sind alle im Gehäuse befestigt.

6. Der Depilator nach Anspruch 5, wobei das Gehäuse eine erste Führungsöffnung (24) und eine zweite Führungsöffnung (25) definiert; die erste Führungsöffnung liegt neben dem Kühlungstreiber; die zweite Führungsöffnung liegt neben dem Haarentfernungsgerät; ein Kühlkanal ist zwischen der ersten Führungsöffnung und der zweiten Führungsöffnung gebildet; der Kühlungstreiber ist konfiguriert, um das externe Kühlmedium im Kühlkanal fließen zu lassen.

7. Der Depilator nach Anspruch 6, wobei der Kühlungshalter (60) im Gehäuse befestigt ist; der erste Kühlkanal und der zweite Kühlkanal sind Teile des Kühlkanals; ein Ende des Kühlungstreibers stößt an die erste Führungsöffnung; eine periphere Seite des Kühlungstreibers stößt an den ersten Kühlkanal und den zweiten Kühlkanal; Enden des ersten Kühlkanals und des zweiten Kühlkanals, die vom Kühlungstreiber entfernt sind, stoßen an die zweite Führungsöffnung.

8. Der Depilator nach Anspruch 7, wobei der Kühlungshalter eine untere Schale (63) und eine obere Schale (64) umfasst, die die untere Schale bedeckt; der Wärmeleitteil ist an einer Seite der oberen Schale befestigt, die von der unteren Schale entfernt ist; ein Teil des Kältekompressionsteils ist zwischen der unteren Schale und der oberen Schale befestigt, und der andere Teil des Kältekompressionsteils durchdringt die obere Schale, um mit dem Wärmeleitteil in Kontakt zu kommen; der erste Kühlkanal und der zweite Kühlkanal sind zwischen der unteren Schale und der oberen Schale gebildet.

9. Der Depilator nach Anspruch 7, wobei der Wärmeleitteil eine Wärmeleitplatte (41) umfasst, die am Kältekompressionsteil angebracht ist, und eine Kühlkörpereinheit (42), die an der Wärmeleitplatte angebracht ist; die Wärmeleitplatte ist am Kühlungshalter befestigt, ein Ende der Wärmeleitplatte, das vom Kältekompressionsteil entfernt ist, versiegelt und bedeckt den zweiten Kühlkanal; die Kühlkörpereinheit ist im zweiten Kühlkanal untergebracht und steht in Kontakt mit einem Teil der Wärmeleitplatte, der den zweiten Kühlkanal bedeckt.

10. Der Depilator nach Anspruch 7, wobei der Depilator weiterhin ein Steuergerät (70) umfasst, das im Gehäuse befestigt ist; das Steuergerät ist elektrisch mit dem Haarentfernungsgerät und dem Kältekompressionsteil gekoppelt und befindet sich auf einer Seite des Haarentfernungsgeräts, die vom Kältekompressionsteil entfernt ist; ein Ende des Gehäuses, das vom Kopfteil entfernt ist, definiert eine dritte Führungsöffnung (26); der Kühlkanal ist weiterhin zwischen der dritten Führungsöffnung und der ersten Führungsöffnung gebildet, sodass das Steuergerät auf dem Kühlkanal positioniert ist und die Wärme des Steuergeräts durch das externe Kühlmedium abgeführt wird.

11. Der Depilator nach Anspruch 10, wobei das Steuergerät eine Leiterplatte (71), einen Prozessor und einen Kondensator (73) umfasst; der Prozessor ist auf der Leiterplatte angeordnet; der Kondensator ist elektrisch mit der Leiterplatte verbunden; die Leiterplatte ist elektrisch mit dem Kältekompressionsteil und dem Haarentfernungsgerät verbunden.

12. Der Depilator nach Anspruch 10, wobei das Gehäuse eine vierte Führungsöffnung (27) definiert, die an die erste Führungsöffnung angrenzt; die vierte Führungsöffnung liegt direkt gegenüber dem Wärmeleitteil; der Kühlkanal ist weiterhin zwischen der vierten Führungsöffnung und der ersten Führungsöffnung gebildet, sodass die Wärme des Wärmeleitteils durch das externe Kühlmedium im Kühlkanal abgeführt wird.

13. Der Depilator nach einem der Ansprüche 1 bis 12, wobei der Kältekompressionsteil ein Kältekompressionsglied (119) und ein Kühlungsglied (118) umfasst, das das Kältekompressionsglied kühlt; das Kältekompressionsglied ist so konfiguriert, dass es mit der Haut des Benutzers in Kontakt kommt, um eine Kältekompression durchzuführen; der Wärmeleitteil ist in Kontakt mit dem Kühlungsglied, um die Wärme des Kühlungsgliedes zu absorbieren; und der Beleuchtungsteil umgibt das Kältekompressionsglied.

14. Der Depilator nach einem der Ansprüche 7 bis 12, wobei das Haarentfernungsgerät eine Lampe (31), eine Lampenhalterung (32) und eine Reflektierende Platte (33) umfasst; die Lampenhalterung ist im Kühlungshalter befestigt und nahe am Kältekompressionsteil; die Lampe und die reflektierende Platte sind an der Lampenhalterung befestigt; die reflektierende Platte befindet sich auf einer Seite der Lampe, die vom Kältekompressionsteil entfernt ist; die reflektierende Platte konzentriert das Licht der Lampe auf das Kältekompressionsteil.

## Revendications

1. Un dépilateur (100), où le dépilateur comprenant une partie tête (10) et une partie tenue à la main (20) accouplée à la partie tête; la partie tête comprend une partie de compression froide (11) et une partie éclairante (12) disposée autour de la partie de compression froide;
la partie éclairante est configurée pour émettre de la lumière pour soigner la peau d'un utilisateur; la partie tenue à la main comprend un dispositif d'épilation (30), une partie de conduction thermique (40) et une partie d'entraînement de refroidissement (50); le dispositif d'épilation est directement opposé à la partie de compression froide, configuré pour émettre de la lumière vers la partie de compression froide; la partie de conduction thermique est en contact avec la partie de compression froide, pour absorber la chaleur de la partie de compression froide; la partie d'entraînement de refroidissement est fixée sur un côté du dispositif d'épilation à l'écart de la partie de compression froide, et configurée pour entraîner un milieu de refroidissement externe à circuler à travers une partie de la partie de conduction thermique éloignée de la partie de compression froide, de sorte que la chaleur de la partie de conduction thermique soit emportée par le milieu de refroidissement externe,
où le dépilateur (100) étant **caractérisé en ce qu'**il comprend en outre un support de refroidissement (60),
où le support de refroidissement (60) étant solidement accouplé à la partie d'entraînement de refroidissement (50) et définissant un premier canal de refroidissement (61) et un deuxième canal de refroidissement (62), qui est séparé du premier canal de refroidissement (61);
le dispositif d'épilation (30) est fixé dans le premier canal de refroidissement (61); et
une partie de la partie de conduction thermique (40) éloignée de la partie de compression froide (11) est fixée dans le deuxième canal de refroidissement (62).

2. Le dépilateur selon la revendication 1, dans lequel la partie éclairante comprend un substrat de source lumineuse (121) disposé autour de la partie de compression froide et une pluralité de perles de lampe (122) disposées sur le substrat de source lumineuse; la pluralité de perles de lampe est disposée à intervalles équidistants autour de la partie de compression froide dans une direction circonférentielle.

3. Le dépilateur selon la revendication 2, dans lequel la partie tête comprend en outre un couvercle de lumière (13); le couvercle de lumière recouvre la pluralité de perles de lampe, et définit un trou de montage (131) pour un ajustement avec jeu avec la partie de compression froide ; une surface de la partie de compression froide éloignée du dispositif d'épilation est affleurante avec une surface du couvercle de lumière éloignée des perles de lampe.

4. Le dépilateur selon la revendication 3, dans lequel le couvercle de lumière est un couvercle transparent ou semi-transparent.

5. Le dépilateur selon la revendication 3, dans lequel la partie tenue à la main comprend un boîtier (21); une extrémité du boîtier est appuyée contre le couvercle de lumière; la partie de compression froide, le dispositif d'épilation, la partie d'entraînement de refroidissement et la partie de conduction thermique sont tous fixés dans le boîtier.

6. Le dépilateur selon la revendication 5, dans lequel le boîtier définit une première ouverture de guidage (24) et une deuxième ouverture de guidage (25); la première ouverture de guidage est adjacente à la partie d'entraînement de refroidissement; la deuxième ouverture de guidage est adjacente au dispositif d'épilation; un canal de refroidissement est formé entre la première ouverture de guidage et la deuxième ouverture de guidage; la partie d'entraînement de refroidissement est configurée pour entraîner le milieu de refroidissement externe à circuler dans le canal de refroidissement.

7. Le dépilateur selon la revendication 6, dans lequel le support de refroidissement (60) est fixé dans le boîtier; les premier et deuxième canaux de refroidissement sont des parties du canal de refroidissement; une extrémité de la partie d'entraînement de refroidissement est appuyée contre la première ouverture de guidage; un côté périphérique de la partie d'entraînement de refroidissement est appuyé contre les premier et deuxième canaux de refroidissement ; les extrémités des premier et deuxième canaux de refroidissement éloignées de la partie d'entraînement de refroidissement sont appuyées contre la deuxième ouverture de guidage.

8. Le dépilateur selon la revendication 7, dans lequel le support de refroidissement comprend une coque inférieure (63) et une coque supérieure (64) recouvrant la coque inférieure; la partie de conduction thermique est fixée à un côté de la coque supérieure éloigné de la coque inférieure; une partie de la partie de compression froide est fixée entre la coque inférieure et la coque supérieure, et l'autre partie de la partie de compression froide traverse la coque supérieure pour entrer en contact avec la partie de conduction thermique; les premier et deuxième canaux de refroidissement sont formés entre la coque inférieure et la coque supérieure.

9. Le dépilateur selon la revendication 7, dans lequel la partie de conduction thermique comprend une plaque de conduction thermique (41) attachée à la partie de compression froide et un ensemble dissipateur thermique (42) attaché à la plaque de conduction thermique; la plaque de conduction thermique est fixée sur le support de refroidissement, une extrémité de la plaque de conduction thermique éloignée de la partie de compression froide scelle et couvre le deuxième canal de refroidissement; l'ensemble dissipateur thermique est logé dans le deuxième canal de refroidissement et est en contact avec une partie de la plaque de conduction thermique couvrant le deuxième canal de refroidissement.

10. Le dépilateur selon la revendication 7, dans lequel le dépilateur comprend en outre un dispositif de commande (70) fixé dans le boîtier; le dispositif de commande est couplé électriquement au dispositif d'épilation et à la partie de compression froide, et est situé sur un côté du dispositif d'épilation éloigné de la partie de compression froide; une extrémité du boîtier éloignée de la partie tête définit une troisième ouverture de guidage (26); le canal de refroidissement est en outre formé entre la troisième ouverture de guidage et la première ouverture de guidage, de sorte que le dispositif de commande est situé sur le canal de refroidissement et la chaleur du dispositif de commande est emportée par le milieu de refroidissement externe.

11. Le dépilateur selon la revendication 10, dans lequel le dispositif de commande comprend une carte de circuit (71), un processeur et un condensateur (73); le processeur est disposé sur la carte de circuit; le condensateur est électriquement couplé à la carte de circuit; la carte de circuit est électriquement couplée à la partie de compression froide et au dispositif d'épilation.

12. Le dépilateur selon la revendication 10, dans lequel le boîtier définit en outre une quatrième ouverture de guidage (27) adjacente à la première ouverture de guidage; la quatrième ouverture de guidage est directement opposée à la partie de conduction thermique ; le canal de refroidissement est en outre formé entre la quatrième ouverture de guidage et la première ouverture de guidage, de sorte que la chaleur de la partie de conduction thermique est emportée par le milieu de refroidissement externe dans le canal de refroidissement.

13. Le dépilateur selon l'une quelconque des revendications 1 à 12, dans lequel la partie de compression froide comprend un élément de compression froide (119) et un élément de refroidissement (118) qui refroidit l'élément de compression froide; l'élément de compression froide est configuré pour entrer en contact avec la peau de l'utilisateur pour effectuer une compression froide; la partie de conduction thermique est en contact avec l'élément de refroidissement pour absorber la chaleur de l'élément de refroidissement; et, la partie éclairante entoure l'élément de compression froide.

14. Le dépilateur selon l'une quelconque des revendications 7 à 12, dans lequel le dispositif d'épilation comprend un tube de lampe (31), un support de tube de lampe (32) et une plaque réfléchissante (33); le support de lampe est fixé dans le support de refroidissement, et proche de la partie de compression froide; le tube de lampe et la plaque réfléchissante sont fixés sur le support de tube de lampe; la plaque réfléchissante est située sur un côté du tube de lampe éloigné de la partie de compression froide; la plaque réfléchissante concentre la lumière du tube de lampe sur la partie de compression froide.
